# EUROPÄISCHE PATENTSCHRIFT

(11) **EP 2 911 653 B1**
(45) Veröffentlichungstag und Bekanntmachung des Hinweises auf die Patenterteilung: **28.02.2018**
(21) Anmeldenummer: 13794793.3
(22) Anmeldetag: 23.10.2013
(51) Int. Cl.: A61K 9/28, A61K 47/34

(54) **FILMBESCHICHTUNGSZUSAMMENSETZUNG**
FILM COATING COMPOSITION
COMPOSITION DE FILM D'ENROBAGE

(30) Priorität: 23.10.2012 AT 504702012
(43) Veröffentlichungstag der Anmeldung: 02.09.2015
(73) Patentinhaber: Gebro Holding GmbH, 6391 Fieberbrunn (AT)
(72) Erfinder: HÄUSLER, Franz, 83435 Bad Reichenhall (DE); ORSI, Harald, I-39057 Eppan (IT)
(74) Vertreter: Wildhack & Jellinek
(86) Internationale Anmeldenummer: PCT/AT2013/050206
(87) Internationale Veröffentlichungsnummer: WO 2014/063178

(56) Entgegenhaltungen:
- EP-B1- 1 208 143
- WO-A1-03/070224
- WO-A1-2010/132204
- WO-A1-2012/061703

## Beschreibung

Die Erfindung liegt auf dem Gebiet der Filmbeschichtung von Trägermaterialien und bezieht sich auf eine Filmbeschichtungszusammensetzung gemäß dem Oberbegriff des Anspruchs 1.

Wesentlich für Filmbeschichtungen, insbesondere für Tabletten im pharmazeutischen Bereich, ist der Schutz vor externer Feuchtigkeit bzw. die Resistenz gegen hohe Feuchtigkeit und hohe Temperatur. Vor allem für heiße und feuchte Länder der Klimazonen III und IV sind Lacke auf Hydroxypropylmethylcellulose (HPMC)-Basis oft nicht geeignet, da ihr Aussehen nach einer Lagerdauer von 6 Monaten bei 40°C und einer relativen Luftfeuchtigkeit von 75 % nicht mehr, wie gefordert, der Spezifikation entspricht.

Unter diesen Gesichtspunkten ist das Polymer Polyvinylalkohol (PVA) als filmbildendes Polymer vorteilhaft, da es die Tablettenkerne effektiv vor externer Feuchtigkeit schützt und da der Lack selbst unter Bedingungen hoher Temperatur und hoher Luftfeuchtigkeit sein Aussehen nicht verändert und seine Integrität beibehält. Aus dem Stand der Technik sind entsprechend diverse Filmbeschichtungen auf Basis von Polyvinylalkohol (PVA) bekannt.

Ein bekannter Nachteil bei der Verwendung von Polyvinylalkohol als Coatingpolymer ist allerdings dessen starke Klebrigkeit während der Verarbeitung. Gerade reines Polyvinylalkohol (PVA), das besonders gute Eigenschaften hinsichtlich des Schutzes vor Feuchtigkeit und Temperatur hätte, ist aufgrund der viel zu hohen Klebeneigung praktisch nicht verarbeitbar. Es wurde deshalb auf verschiedene Weise versucht, diese Klebetendenz zu verringern.

So gab es im Stand der Technik Versuche, die Klebeneigung von Polyvinylalkohol (PVA) durch Zusätze von Talk- und Hydroxypropylcellulose zu verringern.

Weiters ist im US 5,885,617 eine Filmbeschichtungszusammensetzung enthaltend Polyvinylalkohol (PVA) und Lecithin beschrieben. Diese Filmüberzüge weisen akzeptable feuchtigkeitabweisende Eigenschaften auf, besitzen allerdings eine noch immer hohe Klebrigkeit und erlauben dementsprechend nur eine langsame Sprührate.

In der EP 1 208 143 A1 ist weiters eine Zusammensetzung beschrieben, die Polyvinylalkohol (PVA), Polyethylenglycol (PEG) und Talkum umfasst. Dadurch wurden die Glasübergangstemperatur und die Sprödbrüchigkeit des Polymers gesenkt. Auch konnte mit dieser Zusammensetzung die Klebeneigung des Polyvinylalkohol verringert werden.

Ebenso wird im Stand der Technik zur Verbesserung der Klebeneigung die Möglichkeit beschrieben, ein Copolymerisat aus Polyvinylalkohol (PVA) und Polyethylenglycol (PEG) zu bilden.

Filmbeschichtungszusammensetzungen aus PVA-haltigen Copolymeren, z.B. aus PVA-PEG-Copolymeren, oder aus Polymermischungen mit PVA und anderen polymeren Filmbildnern, z.B. aus PVA-PEG Copolymeren mit PVA, wie sie beispielsweise auch aus der WO 2003070224 A1 bekannt sind, sind allerdings im Vergleich zu reinem Polyvinylalkohol (PVA) als polymerem Filmbildner andere, unterschiedliche Polymere mit anderen Eigenschaften.

All diese Maßnahmen waren jedoch nicht in allen gewünschten Aspekten zufriedenstellend.

Es ist damit Aufgabe der Erfindung eine Filmbeschichtungszusammensetzung auf Basis von Polyvinylalkohol (PVA) als einzigem polymerem Filmbildner zu schaffen, die einen Überzug bildet, der sowohl eine möglichst geringe Klebeneigung bei der Verarbeitung als auch gute feuchtigkeitsabweisende Eigenschaften besitzt.

Weiters soll der Überzug gute mechanische Eigenschaften, insbesondere eine ausreichende Elastizität, um unvermeidliche Quellungs- und Schrumpfungsvorgänge des Kerns während der Lagerung auszugleichen, sowie eine gute Filmhaftung und Zugfestigkeit besitzen und außerdem eine glatte Oberfläche und einen klaren Glanz aufweisen.

Außerdem soll der Überzug für den Einsatz beim Menschen oder bei Tieren, insbesondere für pharmazeutische Präparate, geeignet sein.

Weiters ist es Aufgabe der Erfindung ein Verfahren zur Beschichtung von Trägermaterialien zu schaffen, bei dem die Filmbeschichtungszusammensetzung in einem Sprühverfahren mit hoher Sprührate auf die Trägermaterialien aufgebracht werden kann.

Diese Aufgaben werden durch die kennzeichnenden Merkmale der unabhängigen Ansprüche gelöst.

Erfindungsgemäß ist bei der Filmbeschichtungszusammensetzung der eingangs erwähnten Art vorgesehen, dass als der zumindest eine Weichmacher ein nichtionisches Ethylenoxid-Propylenoxid-Blockcopolymer bzw. Poloxamer der allgemeinen Formel I 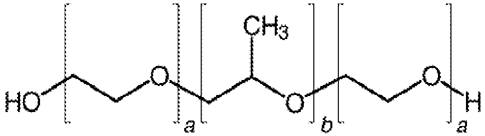 enthalten ist.

Überraschend hat sich gezeigt, dass die Klebrigkeit des Films auf diese Weise weiter reduziert und deutlich unter die Werte eines mit Polyethylenglycol (PEG) versetzten PVA-Films gesenkt werden kann. Gleichzeitig sind aber die feuchtigkeitsabweisenden Eigenschaften eines solchen Überzugs entgegen der Erwartung ebenfalls sehr gut, obwohl die hydrophilen Eigenschaften von Weichmachern oft gerade das Gegenteil bewirken. Die Schutzwirkung der erfindungsgemäßen Filmbeschichtungszusammensetzung vor Feuchtigkeit bzw. deren Stabilität bei hoher Luftfeuchtigkeit ist vor allem auch bei hohen Temperaturen, beispielsweise in Ländern der Klimazonen III und IV, in hohem Maße gewährleistet.

Auch die Zugfestigkeit der Filmbeschichtung ist hoch, außerdem besitzt die Filmbeschichtung eine ausreichende Elastizität, um die Quellungs- und Schrumpfungsvorgänge des Kerns während der Lagerung auszugleichen. Überraschend hat sich zudem eine qualitativ hochwertige, glatte, und auch bei hoher Feuchtigkeit und Temperatur glatt bleibende Filmoberfläche ergeben. Die optische Beurteilung der Oberfläche der derart beschichteten Tabletten nach Glanz war ebenfalls positiv.

Bei der erfindungsgemäßen Filmbeschichtungszusammensetzung ist Polyvinylalkohol (PVA) als einziger polymerer Filmbildner enthalten bzw. der einzige polymere Filmbildner des Überzugs. Die Filmbeschichtungszusammensetzung ist also nur mit einem einzigen filmbildenden Polymer bzw. Filmbildner, nämlich Polyvinylalkohol (PVA), gebildet und nicht z.B. aus PVA-haltigen Copolymeren, z.B. PVA-PEG-Copolymeren, oder aus Polymermischungen mit PVA und weiteren polymeren Filmbildnern, z.B. aus PVA-PEG/PVA Blends.

Die Entdeckung dieser vorteilhaften Eigenschaften kommt unter anderem auch deshalb so überraschend, da Poloxamere in der Literatur vor allem als Tenside beschrieben sind und zur Dispergierung und Emulgierung in der chemisch-technischen Industrie Anwendung finden, sowie in diversen medizinischen und biologischen Applikationen eingesetzt werden. Die Verwendung von Poloxameren als Weichmacher für pharmazeutisch einsetzbare Coatingpolymere, insbesondere für das Polymer Polyvinylalkohol (PVA), zur Ausbildung eines pharmazeutisch akzeptablen Films war bislang nicht bekannt.

Alle Bestandteile der erfindungsgemäßen Filmbeschichtungszusammensetzung erfüllen vorzugsweise die Spezifikationen und Kriterien des Europäischen Arzneibuchs und werden gemäß diesen Anleitungen eingesetzt.

Poloxamere sind synthetische Block-Copolymere, die aus Ethylenoxid und Propylenoxid polymerisiert werden. Die Herstellung von Poloxameren ist seit den 1950er Jahren bekannt, beispielsweise aus der US 3,740,421. In pharmazeutischer Qualität sind im Wesentlichen fünf Poloxamer Typen erhältlich.

| Poloxamer | Molekulargewicht | Anteil Ethylenoxid |
|---|---|---|
| 124 | ca. 2 200 | ca. 47 % |
| 188 | ca. 8 600 | ca. 82 % |
| 237 | ca. 7 800 | ca. 72 % |
| 338 | ca. 15 000 | ca. 83 % |
| 407 | ca. 12 200 | ca. 73 % |

Die Poloxamere 188, 237, 338 und 407 sind fest und pulverförmig und in Wasser oder Ethanol löslich.

Besonders gute weichmachende Eigenschaften werden erzielt, wenn Poloxamere eingesetzt werden, die eine mittlere relative Molekülmasse von 5000 bis 18000, insbesondere von 6500 bis 9600, aufweisen. Weiters ist es vorteilhaft, wenn die mittlere relative Molekülmasse des Polypropylenoxid-Blocks im Bereich von 1500 bis 4500 liegt. Die mittlere relative Molekülmasse ist dabei gemessen nach der Vorschrift des Europäischen Arzneibuchs Ph. Eur. 7. Ausgabe, Grundwerk 2011, Poloxamere 7.0/1464.

Als gut geeignet haben sich Poloxamere erwiesen, bei denen der relative Massenanteil der Ethylenoxid-Einheiten im Bereich von 70 % bis 90 % liegt. Die Ethylenoxid-Einheiten werden dabei gemessen nach der Vorschrift des Europäischen Arzneibuchs Ph. Eur. 7. Ausgabe, Grundwerk 2011, Poloxamere 7.0/1464 sowie Punkt 2.2.33.

Es hat sich weiters als vorteilhaft herausgestellt, vor allem für die Erzielung der gewünschten weichmachenden Eigenschaften, wenn im Poloxamer der allgemeinen Formel I, a den Wert 50 bis 150 und b den Wert 15 bis 60 hat und a + b ≥ 60, insbesondere ≥ 65, ist. Besonders bevorzugt hat a dabei den Wert 60 bis 110 und b den Wert 20 bis 60 und ist a + b ≥ 80.

Eine vorteilhafte Filmbeschichtungszusammensetzung ist dadurch gekennzeichnet, dass Poloxamer 188, Poloxamer 237, Poloxamer 338 und/oder Poloxamer 407 oder eine Mischung davon enthalten ist. Poloxamer 188 und Poloxamer 407 sind dabei besonders gut als Weichmacher geeignet. Vor allem Poloxamer 188 neigt am wenigsten zur Kristallisation im Film.

Vorteilhafterweise ist Poloxamer in der Filmbeschichtungszusammensetzung in einem Gewichtsanteil von 5 % bis 35 %, vorzugsweise 8 % bis 20 %, bezogen auf das Gesamtgewicht der trockenen Filmbeschichtungszusammensetzung enthalten.

Polyvinylalkohol (PVA) ist der einzige polymere Filmbildner des Überzugs. Der filmbildende Anteil des Überzugs besteht damit nur aus reinem PVA Homopolymer als einzigem polymeren Filmbildner. Ein weiteres Polymer mit filmbildenden Eigenschaften existiert nicht. Polyvinylakohol (PVA) liegt also weder in Form eines Copolymers, sondern als Homopolymer, noch in Mischung mit einem anderen filmbildenden Polymer vor. Die erfindungsgemäße Filmbeschichtungszusammensetzung ist somit frei von anderen Filmbildnern bzw. filmbildenden Polymeren außer PVA.

Nicht ausgeschlossen ist dadurch allerdings die Verwendung anderer, nicht filmbildender Zusatzstoffe bzw. Hilfsstoffe mit Polymereigenschaften im Überzug, insbesondere und exemplarisch die Verwendung der polymeren Weichmacher Poloxamere. Derartige Additive bzw. Hilfsstoffe haben keine filmbildenden Eigenschaften und zählen nicht zu den Filmbildnern im Sinne der Erfindung.

Polyvinyalkohol wird durch Polymerisation von Vinylacetat mit anschließender partieller hydrolytischer Abspaltung der Acetylgruppe gewonnen. Dadurch enthält Polyvinylalkohol (PVA) der allgemeinen Formel II 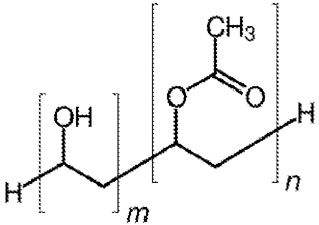 noch signifikante Mengen an Acetylgruppen bzw. teilweise hydrolysiertes Polyvinylacetat. Das Verhältnis der Acetylgruppen (n) zu den Hydroxylgruppen (m) n/m ist 0,35 oder kleiner als 0,35. Die mittlere relative Molekülmasse von Polyvinylalkohol liegt zwischen 20.000 und 150.000, die Viskosität zwischen 3 und 70 mPa*s und die Esterzahl beträgt max. 280. Die Parameter werden ermittelt gemäß dem Europäischen Arzneibuch Ph. Eur. 7. Ausgabe, Grundwerk 2011, Punkt 7.0/1961, S. 4191.

Es hat sich für die Erzielung der gewünschten, insbesondere für die feuchtigkeitsabweisenden und mechanischen, Eigenschaften, als vorteilhaft erwiesen, wenn das Verhältnis der Acetylgruppen (n) zu den Hydroxylgruppen (m) n/m ≤ 0,30 ist und vorzugsweise im Bereich von 0,10 bis 0,20, besonders bevorzugt im Bereich von 0,12 bis 0,16, liegt.

Vorteilhafterweise weist der Polyvinylalkohol (PVA) eine Viskosität von 3 bis 10 mPa*s, besonders bevorzugt von ca. 4 bis 6 mPa*s, insbesondere etwa 5 mPa*s auf. Die Viskosität wird dabei bei einer 4 %igen Lösung gemessen und zwar nach der Vorschrift des Europäischen Arzneibuchs Ph. Eur. 7. Ausgabe, Grundwerk 2011, Punkt 2.2.49.

Eine mechanisch vorteilhafte, wenig klebende und gut feuchtigkeitsabweisende Filmbeschichtungszusammensetzung ergibt sich, wenn Polyvinylalkohol (PVA) in einem Gewichtsanteil von 15 % bis 55 %, vorzugsweise 33 % bis 47 %, bezogen auf das Gesamtgewicht der trockenen Filmbeschichtungszusammensetzung enthalten ist.

Ab einem PVA-Anteil von etwa 20 % beginnt sich die Klebrigkeit bei der Verarbeitung störend bemerkbar zu machen. Eine weitere besonders vorteilhafte Ausführungsform sieht daher zur Reduktion der Klebrigkeit vor, dass als Füllstoff Cellulose, Lactose, Calciumcarbonat, Kaolin und/oder Talk enthalten ist.

Besonders bevorzugt ist Talk enthalten. Talk reduziert die Klebeneigung des Überzugs und verbessert als Gleitmittel bzw. Gleitregulierungsmittel auch das Rutschen der zu beschichtenden Kerne im Kessel. Talk hat damit gleitfördernde und formtrennende Eigenschaften und trägt auch dazu bei, den endgültigen Überzug glatter zu gestalten. Talk in einem Anteil zwischen 10 und 50 Gew.-%, vorzugsweise zwischen 25 und 40 Gew.-%, erhöht überraschend auch die Zugfestigkeit des Filmüberzugs, so dass ein stärkerer Film entsteht. Dies war unerwartet, da eher davon auszugehen war, dass die Zugfestigkeit eines Filmüberzugs mit zunehmendem Anteil eines unlöslichen Bestandteils, wie Talk, tendenziell geringer sein würde.

Weiters ist vorteilhafterweise vorgesehen, dass ein Pigment oder Trübungsmittel, insbesondere Titandioxid oder Eisenoxid, in einem Gewichtsanteil von 0 % bis 40 %, vorzugsweise 5 % bis 25 %, bezogen auf das Gesamtgewicht der trockenen Filmbeschichtungszusammensetzung enthalten ist. Als Pigment/Trübungsmittel können beliebige, für Lebensmittel zugelassene Farben, Trübungsmittel oder Farbstoffe eingesetzt werden. So können zum Beispiel als Pigment/Trübungsmittel Aluminiumpigmente, Eisenoxide, Titandioxid oder natürliche Farben eingesetzt werden.

Außer Füllstoffen können in der Filmbeschichtungszusammensetzung noch weitere Hilfsstoffe enthalten sein, z.B. Tenside, Geschmacksstoffe, Aufheller, Stoffe, um den Lack hydrophiler oder lipophiler zu machen, etc..

Eine besonders bevorzugte Filmbeschichtungszusammensetzung enthält Polyvinylalkohol (PVA) in einem Gewichtsanteil von 30 % bis 50 %, Talkum in einem Gewichtsanteil von 20 % bis 40 %, Poloxamer 188 und/oder Poloxamer 237 in einem Gewichtsanteil von 5 % bis 20 % und gegebenenfalls Titandioxid und/oder Farbpigmente in einem Gewichtsanteil von 5 % bis 20 %, wobei die Gewichtsanteile auf das Gesamtgewicht der trockenen Filmbeschichtungszusammensetzung bezogen sind.

Eine ebenso bevorzugte und einfache Filmbeschichtungszusammensetzung besteht abschließend aus diesen Bestandteilen und keinen anderen.

Die Erfindung betrifft in einem weiteren Aspekt ein Trägermaterial, insbesondere ein Arzneimittel, Nahrungsergänzungsmittel, Lebensmittel, Kosmetika, Medizinprodukt oder landwirtschaftliches Produkt, vorzugsweise in Form von Tabletten, Pellets, Granulaten, Saatgut oder Kristallen, das an seiner Außenfläche mit der erfindungsgemäßen Filmbeschichtungszusammensetzung oder einer vorteilhaften Ausgestaltung derselben beschichtet ist.

Die Erfindung betrifft in noch einem weiteren Aspekt eine flüssige Filmbeschichtungsdispersion bzw. -suspension zur Verwendung zur Beschichtung von Trägermaterialien, insbesondere Arzneimitteln, Nahrungsergänzungsmitteln, Lebensmitteln, Kosmetika, Medizinprodukten oder landwirtschaftlichen Produkte, vorzugsweise in Form von Tabletten, Pellets, Granulaten, Saatgut oder Kristallen, umfassend die Bestandteile der erfindungsgemäßen trockenen Filmbeschichtungszusammensetzung, dispergiert und teilweise gelöst in einem flüssigen Medium.

In diesem Zusammenhang ist es vorteilhaft, wenn Wasser als flüssiges Medium verwendet wird. Hierbei ist es vorteilhaft, wenn der Anteil der nicht wässrigen Bestandteile der Filmbeschichtungsdispersion zwischen 10 und 30 Gew.-%, insbesondere zwischen 15 % und 25 %, bezogen auf die gesamte Dispersion liegt. Analog kann auch ein Wasser/Alkohol Gemisch mit einem Alkoholgehalt von 1 bis 50 Vol %, wobei die Alkohole Ethanol und/oder Isopropanol sind, als flüssiges Medium eingesetzt werden. Hierbei ist es vorteilhaft, wenn der Anteil der nicht wässrigen und nicht alkoholischen Bestandteile der Filmbeschichtungsdispersion zwischen 10 und 30 Gew.-%, insbesondere zwischen 15 % und 25 %, bezogen auf die gesamte Dispersion liegt. Unter den nicht wässrigen bzw. nicht alkoholischen Bestandteilen wird die Menge der zum flüssigen Medium zugegebenen Lacktrockensubstanz verstanden bzw. die im flüssigen Medium dispergierten und gelösten Bestandteile der Filmbeschichtungszusammensetzung.

Unter dem Begriff "Anteil der nicht wässrigen Bestandteile der Filmbeschichtungsdispersion" werden also alle anderen Bestandteile außer Wasser verstanden. Unter dem Begriff "Anteil der nicht wässrigen bzw. nicht alkoholischen Bestandteile" werden alle anderen Bestandteile außer Wasser und Alkohol verstanden.

Die Erfindung betrifft in einem weiteren Aspekt die Verwendung von Ethylenoxid-Propylenoxid-Blockpolymeren bzw. Poloxameren, insbesondere der beschriebenen Art, als Weichmacher für Polyvinylalkohol (PVA), insbesondere bei der Herstellung einer Filmbeschichtung für Arzneimitteltabletten auf Basis von Polyvinylalkohol (PVA).

Die Erfindung betrifft in noch einem weiteren Aspekt ein Verfahren zum Beschichten von Trägermaterialien, insbesondere Arzneimitteln, Nahrungsergänzungsmitteln, Lebensmitteln, Kosmetika, Medizinprodukten oder landwirtschaftlichen Produkte od. dgl., vorzugsweise in Form von Tabletten, Pellets, Granulaten, Saatgut oder Kristallen, mit einem Filmüberzug, umfassend die folgenden Verfahrensschritte:
- Mischen der Bestandteile der erfindungsgemäßen trockenen Filmbeschichtungszusammensetzung mit Wasser und/oder Alkohol unter Ausbildung einer flüssigen Beschichtungsdispersion bzw. -suspension,
- Aufbringen, vorzugsweise Aufsprühen, einer wirksamen Menge dieser Dispersion auf die Trägermaterialien zur Bildung eines Filmüberzugs auf den äußeren Flächen der Trägermaterialien, und
- Trocknen des Filmüberzugs auf den Trägermaterialien.

Dieses Verfahren löst dabei ebenfalls die eingangs gestellt Aufgabe und gewährleistet, dass die Filmbeschichtungszusammensetzung rasch und mit einer hohen Sprührate auf die Trägermaterialien aufgesprüht werden kann.

Bei der Herstellung der Coatinglösung ist es vorteilhaft, warmes Wasser zu verwenden und ausreichend lange zu rühren. Dazu sollte der Polyvinylalkohol in einer geeigneten Korngröße und einer geeigneten Kornform vorliegen, die das Polyvinylalkoholpulver einerseits gut benetzbar und andererseits gut löslich macht.

Besonders vorteilhafte, nicht einschränkend zu verstehende Beispiele für die erfindungsgemäße Filmbeschichtungszusammensetzung, die Filmbeschichtungsdispersion sowie für damit beschichtete Trägermaterialien, sowie deren Herstellung werden im Folgenden beschrieben:

### Beispiele für trockene Filmbeschichtungszusammensetzungen:

Die folgenden Formulierungsbeispiele PVA-haltiger Filmcoating-Überzüge umfassen jeweils nur die erfindungsgemäße Filmbeschichtungszusammensetzung der lösungsmittelfreien Lacktrockensubstanz. Die Angaben in Gewichtsprozent beziehen sich entsprechend auf das Gesamtgewicht (100 %) der trockenen Mischung.

Besonders bevorzugt sind die Rezepturen der Formulierungen 1 und 2 sowie alle Rezepturen, die innerhalb der von diesen Rezepturen aufgespannten Konzentrationsbereichen liegen.

**Formulierung 1:**

| | |
|---|---|
| Polyvinylalkohol (PVA) | 45 % |
| | |
| Poloxamer 188 | 10 % |
| Talk | 30 % |
| Titandioxid | 15 % |

**Formulierung 2:**

| | |
|---|---|
| Polyvinylalkohol (PVA) | 35 % |
| | |
| Poloxamer 188 | 15 % |
| Talk | 35 % |
| Titandioxid | 15 % |

**Formulierung 3:**

| | |
|---|---|
| Polyvinylalkohol (PVA) | 50 % |
| | |
| Poloxamer 188 | 8 % |
| Talk | 35 % |
| Titandioxid | 12 % |

**Formulierung 4:**

| | |
|---|---|
| Polyvinylalkohol (PVA) | 25 % |
| | |
| Poloxamer 407 | 20 % |
| Talk | 40 % |
| Titandioxid | 15 % |

**Formulierung 5:**

| | |
|---|---|
| Polyvinylalkohol (PVA) | 40 % |
| | |
| Poloxamer 407 | 13 % |
| Talk | 33 % |
| Titandioxid | 14 % |

**Formulierung 6:**

| | |
|---|---|
| Polyvinylalkohol (PVA) | 40 % |
| | |
| Poloxamer 188 | 13 % |
| Talk | 33 % |
| Titandioxid | 6 % |
| Eisenoxid Farbpigmente | 8 % |

Um ausgehend von diesen Rezepturbeispielen zu beschichteten Filmtabletten zu gelangen, sind noch weitere Schritte erforderlich:

### Herstellungsbeispiel einer Filmbeschichtungssuspension:

Zur Herstellung der erfindungsgemäßen Filmbeschichtungssuspension bzw. Filmcoating-Suspension werden die Bestandteile der Filmbeschichtungszusammensetzung bzw. Lacktrockensubstanz in einem geeigneten Lösungsmittel suspendiert und teilweise gelöst. Als Lösungsmittel wird in den meisten Fällen Wasser verwendet. Wenn das zu beschichtende Material feuchtigkeitsempfindlich ist, kann auch eine Mischung von Wasser und bis zu 50 % Ethanol oder Isopropanol verwendet werden.

In das Lösungsmittel wird die trockene Filmbeschichtungszusammensetzung in einer Konzentration von 10 bis 35 % zugegeben. Im vorliegenden Ausführungsbeispiel 1 beträgt die Konzentration der Filmbeschichtungszusammensetzung (Lacktrockenmasse) etwa 19 %, was einer PVA-Konzentration von ca. 8,6 % entspricht.

Zur Herstellung der Filmsuspension werden die löslichen Bestandteile, also Polyvinylalkohol (PVA) und Poloxamer, in einem Teil des Lösungsmittels unter Rühren gelöst. Die unlöslichen Bestandteile, z.B. Talk, Titandioxid oder Farbpigmente, werden im Rest des Lösungsmittels suspendiert, wobei zur Erzielung einer feinen, klumpenfreien Verteilung vorteilhafterweise ein Homogenisator verwendet wird. Die Lösung der Polymere und die Pigmentsuspension werden anschließend unter Rühren vereinigt.

Eine alternative Ausführungsform der Herstellung besteht darin, dass zunächst alle festen Bestandteile der Filmbeschichtungszusammensetzung in einem geeigneten Mischer oder in einer Mühle oder in einem Granulator homogen gemischt und/oder granuliert werden und dass diese homogene Mischung dann unter Rühren im Lösungsmittel gelöst bzw. suspendiert wird.

### Herstellung einer Filmbeschichtungssuspension auf Basis von Formulierung 1:

Die Herstellung von 20,4 kg einer Filmbeschichtungssuspension bzw. Sprühsuspension mit der spezifischen Filmbeschichtungszusammensetzung gemäß Formulierung 1 kann vorteilhaft auf folgende Weise erfolgen:
13 kg Wasser werden auf eine Temperatur von 50 bis 60 °C erwärmt. Es werden 1,755 kg Polyvinylalkohol zugegeben und es wird 20 min gerührt bis eine klare Lösung entstanden ist. Es werden 0,351 kg Poloxamer 188 zugegen und weitere 20 min gerührt. 1,17 kg Talk und 0,585 kg Titandioxid werden in 3,5 kg Wasser eingerührt und die Suspension wird 15 Minuten homogenisiert. Anschließend werden die Polymerlösung und die Pigmentsuspension vereinigt und es wird weitere 30 min gerührt.

In der so erhaltenen Filmbeschichtungssuspension sind 19 Gew.-% der Filmbeschichtungszusammensetzung enthalten.

### Herstellung von beschichteten Trägermaterialien:

Anschließend wird die so erhaltene flüssige Filmbeschichtungssuspension in einem entsprechend ausgerüsteten Coater auf die Tablettenkerne aufgesprüht. Zur Vermeidung von Sedimentation wird auch während des anschließenden Sprühvorgangs gerührt. Ein Vorteil des Polyvinylalkohols (PVA) besteht dabei in seiner niedrigen Viskosität, was einen hohen Anteil von gelöstem und suspendiertem Material von bis zu 35 % Lacktrockensubstanz in der Sprühsuspension erlaubt. Bei HPMC-Filmen gilt z.B. bereits ein Feststoffgehalt von 15 % als hoch.

Als Tablettenkerne wurden bei den vorliegenden Ausführungsbeispielen Seractil®-Tabletten verwendet. Die erfindungsgemäße Filmbeschichtungssuspension kann aber auch auf eine Vielzahl anderer unterschiedlicher Kerne bzw. Trägermaterialien aufgebracht werden und eignet sich zum Überziehen von Tabletten, Pellets, Granulaten, Kristallen, Samen oder von anderen kleinvolumigen Partikeln. Die Filmbeschichtungssuspension kann für pharmazeutische Produkte, für Nahrungsergänzungsmittel, Medizinprodukte, Kosmetika, Nahrungsmittel, Süßigkeiten oder landwirtschaftliche Produkte verwendet werden.

Während des Sprühvorgangs werden die Kerne kontinuierlich getrocknet, um eine Überfeuchtung zu vermeiden. Die benötigte Auftragsmenge hängt von der gewünschten Funktion und von der Tablettengröße ab. Übliche Auftragsmengen an Lacktrockensubstanz bei Tablettenkernen sind 2 bis 4 % des Kerngewichtes.

### Herstellung von mit der Filmbeschichtungszusammensetzung gemäß Formulierung 1 beschichteten Tabletten:

Unmittelbar vor ihrer Verwendung wird die so erhaltene Filmbeschichtungssuspension nochmals aufgerührt und auch während des Sprühens wird permanent langsam gerührt. Die Filmbeschichtungssuspension wird in einem geeigneten Coater während ca. 1,5 Stunden bei einer Kernbetttemperatur von 35 bis 40 °C auf ca. 100 kg Tablettenkerne, vorliegend Seractil®-Tabletten, aufgesprüht.

Optional kann nach dem Besprühen und Trocknen der Kerne aus optischen Gründen eine äußerliche Polierung mit einer geringen Menge Paraffin, Polyethylenglycol oder Wachs erfolgen.

Es resultieren weiße, glatte, und glänzende Filmtabletten mit einem Filmgewicht von ca. 3,5 % bezogen auf das Kerngewicht. Der gut haftende Film schützt den Tablettenkern wirksam vor dem Einfluss hoher Luftfeuchtigkeit, ohne dabei auch bei hohen Temperaturen sein Erscheinungsbild zu verändern.

In der EP 1 208 143 B1 wird ebenfalls ein Verfahren zur Herstellung von Filmbeschichtungszusammensetzungen und -dispersionen sowie von beschichteten Trägermaterialien beschrieben, das analog auch auf die vorliegenden Ausgangsstoffe anzuwenden ist.

Es wurden verschiedene Vergleichsversuche durchgeführt, um die vorteilhaften Eigenschaften der erfindungsgemäßen Filmbeschichtungszusammensetzungen nachzuweisen. Während der Beschichtung von Tablettenkernen mit verschiedenen Filmbeschichtungszusammensetzungen bei identischen Prozessparametern wurde die Klebetendenz optisch beobachtet, durch Fotos dokumentiert und bewertet. Zur Objektivierung der Befunde wurden die erhaltenen Tabletten mit freiem Auge und unter dem Mikroskop optisch beurteilt und die Neigung zur Zwillingsbildung durch Auszählen der Zwillinge bestimmt.

### Untersuchung der Klebetendenz bei ansteigender Sprührate:

In einer ersten Versuchsreihe wurden zwei Filmtablettenchargen, die gemäß der Erfindung mit einem poloxamerhaltigen PVA-Lack überzogen wurden, mit einer Filmtablettencharge, die mit einem macrogolhaltigen PVA-Lack überzogen wurde, verglichen. Dazu wurden Tablettenkerne nach dem oben beschriebenen Verfahren mit einem Lack der Formulierung 1 (45% PVA / 10% Poloxamer 188 / 30% Talkum / 15% Titandioxid), mit einem alternativen erfindungsgemäßen Lack mit unterschiedlicher Poloxamerkonzentration (45% PVA / 19% Poloxamer 188 / 24% Talkum / 12% Titandioxid) und mit einem analog hergestellten Lack mit dem Weichmacher Macrogol (Polyethylenglycol / PEG) (45% PVA / 19% Macrogol 3350 / 24% Talkum / 12% Titandioxid) überzogen. Die Tabletten wurden dabei unter den oben genannten Standardbedingungen lackiert, wobei jedoch jeweils schrittweise die Sprührate erhöht wurde, um durch zunehmende Befeuchtung der Kerne deren Klebetendenz schrittweise zu erhöhen.

Die beiden poloxamerhaltigen Filme zeigten dabei in beiden Konzentrationen und unabhängig von der Sprührate ein signifikant geringeres Klebeverhalten als der macrogolhaltige Vergleichsfilm. Bereits beim Auftrag der zweiten von fünf verwendeten Sprühraten zeigte sich erstmals ein Unterschied zwischen den Filmen in der Weise, dass ausschließlich der macrogolhaltige Film begann, an der Trommelwand zu kleben. Bei der dritten und vierten Sprührate verstärkte sich diese Tendenz beim macrogolhaltigen Film deutlich. Bei der fünften und höchsten Sprührate brach die Lackierung beim macrogolhaltigen Film wegen massiven Klebens und Zwillingsbildung gänzlich zusammen, während die poloxamerhaltigen Filme weiterhin nicht bzw. nur wenig klebten.

Bei dieser hohen Sprührate wurde auch eine Abhängigkeit der Klebetendenz von der Menge an Poloxamer deutlich. Mehr Poloxamer bedeutete eine geringere Klebetendenz. Dies zeigt, dass der Zusatz von Poloxamer nicht nur einen negativen Effekt des Macrogols vermeidet, sondern, dass das Poloxamer die Klebetendenz aktiv verringert.

### Untersuchung der Klebetendenz bei Veränderung von weiteren Prozessparametern:

Das Ausmaß des Klebens bei der Befilmung hängt nicht nur von der Sprührate ab, sondern auch von weiteren Prozessparametern. Mit einer Verstärkung des Klebeeffektes ist prinzipiell zu rechnen bei einer Erhöhung der Sprührate, bei einer Verringerung der Trommeldrehzahl und bei einer Reduktion der Zulufttemperatur. In einer zweiten Versuchsserie wurde deshalb ein weichmacherfreier Film (50,3% PVA / 33,2% Talkum / 16,5% Titandioxid) verglichen mit einem erfindungsgemäßen Film gemäß Formulierung 1 (45% PVA / 10% Poloxamer 188 / 30% Talkum / 15% Titandioxid) sowie einem Vergleichsfilm mit dem Weichmacher Macrogol (Polyethylenglycol (PEG)) (45% PVA / 10% Macrogol 3350 / 30% Talkum / 15% Titandioxid). Es wurden jeweils die Prozessparameter Sprührate, Trommeldrehzahl und Temperatur variiert und deren Einfluss auf das Klebeverhalten untersucht.

Die Versuche, bei denen die Sprührate erhöht wurde, bestätigten die Ergebnisse der ersten Versuchsreihe. Bei der Charge ohne Weichmacher kam es zu massivem Kleben, wobei der Unterschied bei allen Sprühraten deutlich zu sehen war. Im Vergleich der Formulierung 1 mit dem PEG-haltigen Vergleichsfilm war nach der zweiten von drei Erhöhungen der Sprührate der Vorteil von Poloxamer gegenüber Macrogol als Weichmacher zu erkennen, bei der dritten Erhöhung der Sprührate war er deutlich. Bei dem PEG-haltigen Vergleichsfilm trat nicht nur ein Kleben an der Kesselwand auf, sondern es bildeten sich auch Kernnester im Tablettenbett, was als Verschärfung des Klebeproblems zu werten ist.

Die Versuche, bei denen die Trommeldrehzahl reduziert wurde, wurden in fünf Stufen durchgeführt, bei denen die Trommeldrehzahl schrittweise von 16 U/min auf 8 U/min reduziert wurde. Der Vorteil von Poloxamer gegenüber Macrogol bezüglich der Klebetendenz war bei allen Trommeldrehzahlen erkennbar und wurde mit abnehmender Trommeldrehzahl zunehmend deutlicher. Mit dem weichmacherfreien Lack war bereits bei einer Drehzahl auf 12 U/min eine Befilmung nicht mehr möglich.

Die Versuche, bei denen die Zulufttemperatur reduziert wurde, wurden in der Weise durchgeführt, dass die Zulufttemperatur in fünf Schritten von 60 °C auf 40 °C reduziert wurde. Über den gesamten Versuch zeigte sich ein geringeres Kleben des poloxamerhaltigen Lacks gegenüber dem PEG-haltigen Lack, wobei der Effekt mit abnehmender Temperatur zunehmend deutlicher wurde. Mit dem weichmacherfreien Lack war bereits bei einer Zulufttemperatur von 50 °C ehe Befilmung nicht mehr möglich.

### Optische Beurteilung der Filmtabletten:

Am Ende der beiden Versuchsreihen, in denen die Sprührate reduziert wurde, erfolgte außerdem jeweils auch eine optische Beurteilung der Tabletten:

| | mit Poloxamer | mit Polyethylenglycol | ohne Weichmacher |
|---|---|---|---|
| Visuelle Beurteilung der Filmtabletten | spezifikationskonform, glatter, glänzender, gleichmäßiger Überzug, ohne Risse, Absplitterungen oder andere Defekte | spezifikationskonform, Überzug teilweise rau und ungleichmäßig, ohne Risse, Absplitterungen oder andere Defekte | nicht spezifikationskonform, Überzug rau und uneben, partielle Ablösung des Films |
| Stereomikros kopische Beurteilung der Filmtabletten | nur leicht unebene Oberfläche, sehr gleichmäßiger und fein strukturierter Film | unebene Oberfläche mit kleineren Löchern und Erhebung, gleichmäßiger und fein strukturierter Film | unebene Oberfläche mit größeren Löchern und stärkeren Erhebungen ungleichmäßige Filmstruktur |

Ohne Weichmacher war, unabhängig vom technischen Problem des Klebens am und im Kessel, auch die Qualität des Filmüberzuges mangelhaft. Der Zusatz eines Weichmachers ist auch unter diesem Aspekt nötig. Der poloxamerhaltige Film ergab eine glattere, schönere und gleichmäßigeren Oberfläche der befilmten Tabletten als der macrogolhaltige Vergleichsfilm. Die Verwendung von Poloxamer als Weichmacher hat gegenüber der Verwendung von Macrogol damit auch den Vorteil, dass die resultierenden Filmoberflächen eine bessere Qualität aufweisen bzw. dass eine definierte Filmqualität unter kritischeren Lackierbedingungen erreicht werden kann.

Zusammenfassend ergibt sich damit, dass der Weichmacher Poloxamer die Klebetendenz des PVA-Films, z.B. die Neigung zur Zwillingsbildung bzw. zur Ausbildung von Nestern, stärker reduziert als Macrogol PEG 3350. Poloxamer ermöglicht auch unter sehr ungünstigen Lackierbedingungen die Bildung von schönen, glatten und gleichmäßigen Filmen und ist diesbezüglich Macrogol 3350 überlegen.

Über 24 Monate bei verschiedenen Klimabedingungen durchgeführte Lagerversuche zeigten, dass der erfindungsgemäße Filmüberzug auf den Tabletten eine ausgezeichnete Langzeitstabilität besitzt. Auch bei ungünstigen Lagerbedingungen von beispielsweise 40 °C und 75 % relativer Luftfeuchtgkeit zeigen die Filmtabletten einen lang anhaltenden klaren Glanz, eine minimale Klebeneigung im Packmittel und eine gute Filmhaftung und Elastizität.

Die Langzeitstabilitätstests zeigten überdies, dass der erfindungsgemäße Lack die als Träger verwendeten Tablettenkerne ausgezeichnet vor dem Zutritt von Feuchtigkeit aus der Umgebungsluft schützt. Während Seractil®-Tabletten, die mit einem Standardlack auf Basis von HPMC befilmt sind, nach 6-monatiger Lagerung bei 40 °C / 75 % relativer Feuchte und nach 2-jähriger Lagerzeit bei 30 °C / 65 % relativer Feuchte nicht mehr der Spezifikation entsprechen, bestehen Filmtabletten, die mit dem erfindungsgemäßen Lack überzogen sind, die beiden genannten Prüfungen ohne Probleme.

## Patentansprüche

1. Filmbeschichtungszusammensetzung zur Beschichtung von Trägermaterialien, insbesondere Arzneimitteln, Nahrungsergänzungsmitteln, Lebensmitteln, Kosmetika, Medizinprodukten oder landwirtschaftlichen Produkte, vorzugsweise in Form von Tabletten, Pellets, Granulaten, Saatgut oder Kristallen, enthaltend
- Polyvinylalkohol (PVA) als einzigen Filmbildner,
- zumindest einen pharmazeutisch akzeptablen Füllstoff oder ein Gleitmittel,
- sowie zumindest einen Weichmacher,
**dadurch gekennzeichnet, dass** als Weichmacher ein nichtionisches Ethylenoxid-Propylenoxid-Blockcopolymer bzw. Poloxamer der allgemeinen Formel I 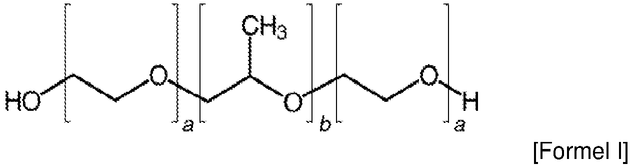 enthalten ist.

2. Filmbeschichtungszusammensetzung nach Anspruch 1, **dadurch gekennzeichnet, dass** das Poloxamer eine mittlere relative Molekülmasse von 5000 bis 18000, insbesondere von 6500 bis 9600, aufweist.

3. Filmbeschichtungszusammensetzung nach einem der vorangehenden Ansprüche, **dadurch gekennzeichnet, dass** die mittlere relative Molekülmasse des Polypropylenoxid-Blocks des Poloxamers im Bereich von 1500 bis 4500 liegt.

4. Filmbeschichtungszusammensetzung nach einem der vorangehenden Ansprüche, **dadurch gekennzeichnet, dass** der relative Massenanteil der Ethylenoxid-Einheiten des Poloxamers im Bereich von 70 % bis 90 % liegt.

5. Filmbeschichtungszusammensetzung nach einem der vorangehenden Ansprüche, **dadurch gekennzeichnet, dass** im Poloxamer der allgemeinen Formel I, a den Wert 50 bis 150 und b den Wert 15 bis 60 hat und a + b ≥ 60, insbesondere ≥ 65, ist, wobei vorzugsweise a den Wert 60 bis 110 und b den Wert 20 bis 60 hat und a + b ≥ 80 ist.

6. Filmbeschichtungszusammensetzung nach einem der vorangehenden Ansprüche, **dadurch gekennzeichnet, dass** Poloxamer 188, Poloxamer 237, Poloxamer 338 und/oder Poloxamer 407 oder eine Mischung davon enthalten ist, wobei Poloxamer 188 und/oder Poloxamer 237 besonders bevorzugt sind.

7. Filmbeschichtungszusammensetzung nach einem der vorangehenden Ansprüche, **dadurch gekennzeichnet, dass** Polyvinylalkohol (PVA) der allgemeinen Formel II 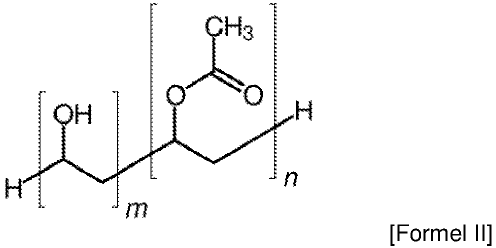 teilweise hydrolysiertes Polyvinylacetat enthält, wobei das Verhältnis der Acetylgruppen (n) zu den Hydroxylgruppen (m) n/m ≤ 0,30 ist, vorzugsweise im Bereich von 0,10 bis 0,20, besonders bevorzugt im Bereich von 0,12 bis 0,16, liegt.

8. Filmbeschichtungszusammensetzung nach einem der vorangehenden Ansprüche, **dadurch gekennzeichnet, dass** der Polyvinylalkohol (PVA) eine Viskosität von 3 bis 10 mPa*s, besonders bevorzugt von ca. 4 bis 6 mPa*s, insbesondere etwa 5 mPa*s, gemessen bei einer 4 %igen Lösung, aufweist.

9. Filmbeschichtungszusammensetzung nach einem der vorangehenden Ansprüche, **dadurch gekennzeichnet, dass** Polyvinylalkohol (PVA) in einem Gewichtsanteil von 15 % bis 55 %, vorzugsweise 33 % bis 47 %, bezogen auf das Gesamtgewicht der trockenen Filmbeschichtungszusammensetzung enthalten ist.

10. Filmbeschichtungszusammensetzung nach einem der vorangehenden Ansprüche, **dadurch gekennzeichnet, dass** als Füllstoff Cellulose, Lactose, Calciumcarbonat, Kaolin, und/oder Talk, in einem Gewichtsanteil von 10 % bis 50 %, vorzugsweise 25 % bis 40 %, bezogen auf das Gesamtgewicht der trockenen Filmbeschichtungszusammensetzung, enthalten ist.

11. Filmbeschichtungszusammensetzung nach einem der vorangehenden Ansprüche, **dadurch gekennzeichnet, dass** Poloxamer in einem Gewichtsanteil von 5 % bis 35 %, vorzugsweise 8 % bis 20 %, bezogen auf das Gesamtgewicht der trockenen Filmbeschichtungszusammensetzung enthalten ist.

12. Filmbeschichtungszusammensetzung nach einem der vorangehenden Ansprüche, **dadurch gekennzeichnet, dass** ein Pigment oder Trübungsmittel, insbesondere Titandioxid oder Eisenoxid, in einem Gewichtsanteil von 0 % bis 40 %, vorzugsweise 5 % bis 25 %, bezogen auf das Gesamtgewicht der trockenen Filmbeschichtungszusammensetzung enthalten ist.

13. Filmbeschichtungszusammensetzung nach einem der vorangehenden Ansprüche, enthaltend, vorzugsweise bestehend aus,
- Polyvinylalkohol (PVA) in einem Gewichtsanteil von 30 % bis 50 %,
- Talk in einem Gewichtsanteil von 20 % bis 40 %,
- Poloxamer 188 oder Poloxamer 237 in einem Gewichtsanteil von 5 % bis 20 %
- und gegebenenfalls Titandioxid und/oder Farbpigmente in einem Gewichtsanteil von 5 % bis 20 %,
wobei die Gewichtsanteile auf das Gesamtgewicht der trockenen Filmbeschichtungszusammensetzung bezogen sind.

14. Trägermaterial, insbesondere ein Arzneimittel, Nahrungsergänzungsmittel, Lebensmittel, Kosmetika, Medizinprodukt oder landwirtschaftliches Produkt, vorzugsweise in Form von Tabletten, Pellets, Granulaten, Saatgut oder Kristallen, an seiner Außenfläche beschichtet mit der Filmbeschichtungszusammensetzung nach einem der Ansprüche 1 bis 13.

15. Flüssige Filmbeschichtungsdispersion zur Verwendung zur Beschichtung von Trägermaterialien, insbesondere Arzneimitteln, Nahrungsergänzungsmitteln, Lebensmitteln, Kosmetika, Medizinprodukten oder landwirtschaftlichen Produkte, vorzugsweise in Form von Tabletten, Pellets, Granulaten, Saatgut oder Kristallen, umfassend die Bestandteile der trockenen Filmbeschichtungszusammensetzung nach einem der Ansprüche 1 bis 13 dispergiert und teilweise gelöst in einem flüssigen Medium.

16. Flüssige Filmbeschichtungsdispersion nach Anspruch 15, dispergiert in Wasser, wobei der Anteil der nicht wässrigen Bestandteile der Filmbeschichtungsdispersion vorzugsweise zwischen 10 und 30 Gew.-%, insbesondere zwischen 15 % und 25 %, liegt oder, dispergiert in einem Wasser/Alkohol Gemisch mit einem Alkoholgehalt von 1 bis 50 Vol %, wobei die Alkohole Ethanol und/oder Isopropanol sind, und wobei der Anteil der nicht wässrigen bzw. nicht alkoholischen Bestandteile der Filmbeschichtungsdispersion vorzugsweise zwischen 10 und 30 Gew.-%, insbesondere zwischen 15 % und 25 %, liegt.

17. Verwendung von Ethylenoxid-Propylenoxid-Blockpolymeren bzw. Poloxameren, insbesondere mit den Merkmalen gemäß einem der Ansprüche 1 bis 6, als Weichmacher für Polyvinylalkohol (PVA), insbesondere bei der Herstellung einer Filmbeschichtung für Arzneimitteltabletten auf Basis von Polyvinylalkohol (PVA).

18. Verfahren zum Beschichten von Trägermaterialien, insbesondere Arzneimitteln, Nahrungsergänzungsmitteln, Lebensmitteln, Kosmetika, Medizinprodukten oder landwirtschaftlichen Produkte, vorzugsweise in Form von Tabletten, Pellets, Granulaten, Saatgut oder Kristallen, mit einem Filmüberzug, umfassend die folgenden Verfahrensschritte:
- Mischen der Bestandteile der trockenen Filmbeschichtungszusammensetzung nach einem der Ansprüche 1 bis 13 mit Wasser und/oder Alkohol unter Ausbildung einer flüssigen Beschichtungsdispersion bzw. -suspension,
- Aufbringen, vorzugsweise Aufsprühen, einer wirksamen Menge dieser Dispersion auf die Trägermaterialien zur Bildung eines Filmüberzugs auf den äußeren Flächen der Trägermaterialien, und
- Trocknen des Filmüberzugs auf den Trägermaterialien.

## Claims

1. A film coating composition for coating carrier materials, particularly drugs, food supplements, foodstuffs, cosmetics, medicinal products or agricultural products, preferably in the form of tablets, pellets, granulates, seeds or crystals, containing
- polyvinyl alcohol (PVA) as the only film former,
- at least one pharmaceutically acceptable filler or lubricant,
- and at least one softener,
**characterised in that** a non-oinic ethylene oxide propylene oxide block copolymer or poloxamer with the general formula I 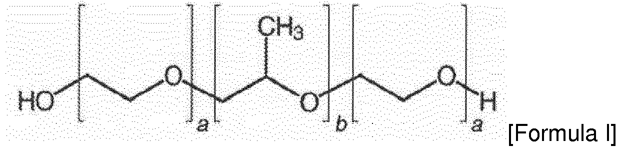 is contained as the softener.

2. The film coating composition according to Claim 1, **characterised in that** the poloxamer has an average relative molecular mass of 5000 to 18000, and in particular of 6500 to 9600.

3. The film coating composition according to any one of the preceding claims, **characterised in that** the average molecular mass of the polypropylene oxide block of the poloxamer is within the range of 1500 to 4500.

4. The film coating composition according to any one of the preceding claims, **characterised in that** the relative mass fraction of the ethylene oxide units of the poloxamer is within the range of 70 % to 90 %.

5. The film coating composition according to any one of the preceding claims, **characterised in that** in the poloxamer with the general Formula I a has the value of 50 to 150 and b has the value of 15 to 60, and that a + b > 60, in particular ≥ 65, wherein preferably a has the value of 60 to 110 and b the value of 20 to 60, and a + b ≥ 80.

6. The film coating composition according to any one of the preceding claims, **characterised in that** it contains poloxamer 188, poloxamer 237, poloxamer 338 and/or poloxamer 407 or a mixture thereof, wherein poloxamer 188 and/or poloxamer 237 is particularly preferable.

7. The film coating composition according to any one of the preceding claims, **characterised in that** polyvinyl alcohol (PVA) with the general Formula II 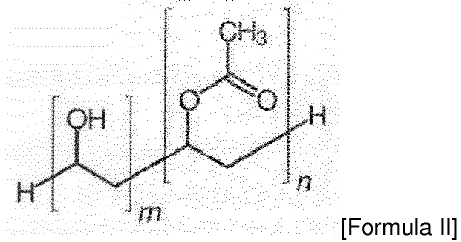 contains partially hydrolysed polyvinyl acetate, wherein the ratio of the acetyl groups (n) to the hydroxyl groups (m) n/m) n/m ≤ 0.30, preferably in the range of 0.10 to 0.20, and in particular preference in the range of 0.12 to 0.16.

8. The film coating composition according to any one of the preceding claims, **characterised in that** the polyvinyl alcohol (PVA) has a viscosity of 3 to 10 mPa*s, in particular preference approx. 4 to 6 mPa*s, and particularly approx. 5 mPa*s, measured in a 4% solution.

9. The film coating composition according to any one of the preceding claims, **characterised in that** it contains polyvinyl alcohol (PVA) in proportion by weight of 15 % to 55 %, preferably 33 % to 47 %, related to the total weight of the dry film coating composition.

10. The film coating composition according to any one of the preceding claims, **characterised in that** it contains cellulose, lactose, calcium carbonate, kaolin and/or talcum as the filler in a proportion by weight of 10 % to 50 %, preferably 25 % to 40 %, related to the total weight of the dry film coating composition.

11. The film coating composition according to any one of the preceding claims, **characterised in that** it contains poloxamer in a proportion by weight of 5 % to 35 %, preferably 8 % to 20 %, related to the total weight of the dry film coating composition.

12. The film coating composition according to any one of the preceding claims, **characterised in that** it contains a pigment or opacifier, in particular titanium dioxide or iron oxide, in a proportion by weight of 0 % to 40 %, preferably 5 % to 25 %, related to the total weight of the dry film coating composition.

13. The film coating composition according to any one of the preceding claims, containing, and preferably consisting of,
- polyvinyl alcohol (PVA) in a proportion by weight of 30 % to 50 %,
- talcum in a proportion by weight of 20 % to 40 %,
- poloxamer 188 or poloxamer 237 in a proportion by weight of 5 % to 20 %
- and, if necessary, titanium dioxide and/or colour pigments in a proportion by weight of 5 % to 20%,
wherein the proportions of weight relate to the total weight of the dry film coating composition.

14. A carrier material, in particular a drug, food supplement, foodstuff, cosmetic, medicinal product or agricultural product, preferably in the form of tablets, pellets, granulates, seeds or crystals, coated on its outside with the film coating composition according to any one of Claims 1 to 13.

15. A liquid film coating dispersion used for coating carrier materials, in particular drugs, food supplements, foodstuffs, cosmetics, medicinal products or agricultural products, preferably in the form of tablets, pallets, granulates, seeds or crystals, comprising the constituents of the dry film coating composition according to any one of Claims 1 to 13, dispersed and partially dissolved in a liquid medium.

16. The liquid film coating dispersion according to Claim 15, dispersed in water, wherein the proportion of the non-aqueous constituents of the film coating composition is preferably between 10 and 30 % by weight, and in particular between 15 % and 25 %, or dispersed in a water/alcohol mixture with an alcohol content of 1 to 50 % by vol., wherein the alcohols are ethanol and/or isopropanol, and wherein the proportion of the non-aqueous or non-alcoholic constituents of the film coating dispersion is preferably between 10 and 30 %, and in particular between 15 % and 25 %.

17. The use of ethylene oxide propylene oxide block polymers or poloxamers, particularly with the features according to any one of Claims 1 to 6, as softeners for polyvinyl alcohol (PVA), particularly in the manufacture of a film coating for drug tablets based on polyvinyl alcohol (PVA).

18. A method for coating carrier materials, particularly drugs, food supplements, foodstuffs, cosmetics, medicinal products or agricultural products, preferably in the form of tablets, pellets, granulates, seeds or crystals, with a film coating, comprising the following method steps:
- mixing of the constituents of the dry film coating composition according to any one of Claims 1 to 13 with water and/or alcohol, forming a liquid coating dispersion or suspension,
- applying, preferably spraying, of an effective quantity of this dispersion, onto the carrier materials for the formation of a film coating on the outer surfaces of the carrier materials, and
- drying the film coating on the carrier materials.

## Revendications

1. Composition de revêtement pelliculaire pour recouvrir des substrats, en particulier des médicaments, des compléments alimentaires, des denrées alimentaires, des cosmétiques, des dispositifs médicaux ou des produits agricoles, de préférence sous forme de comprimés, de pastilles, de granulés, de semences ou de cristaux, contenant
- de l'alcool polyvinylique (PVA) en tant qu'agent filmogène unique,
- au moins une charge ou un lubrifiant pharmaceutiquement acceptable,
- ainsi qu'au moins un plastifiant,
**caractérisée en ce qu'un** copolymère à blocs oxyde d'éthylène-oxyde de propylène non ionique ou poloxamère de formule générale I 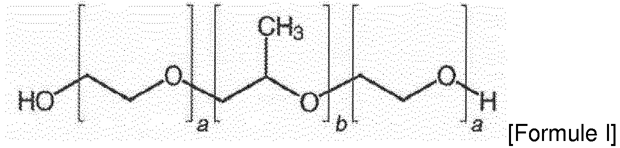 est contenu en tant que plastifiant.

2. Composition de revêtement pelliculaire selon la revendication 1, **caractérisée en ce que** le poloxamère présente une masse moléculaire relative moyenne de 5000 à 18 000, en particulier de 6500 à 9600.

3. Composition de revêtement pelliculaire selon l'une des revendications précédentes, **caractérisée en ce que** la masse moléculaire relative moyenne du bloc oxyde de propylène du poloxamère se situe dans la plage de 1500 à 4500.

4. Composition de revêtement pelliculaire selon l'une des revendications précédentes, **caractérisée en ce que** la fraction massique relative des motifs oxyde d'éthylène du poloxamère se situe dans la plage de 70 % à 90 %.

5. Composition de revêtement pelliculaire selon l'une des revendications précédentes, **caractérisée en ce que**, dans le poloxamère de formule générale I, la valeur de a est de 50 à 150 et la valeur de b est de 15 à 60 et a + b ≥ 60, en particulier ≥ 65, de préférence la valeur de a étant de 60 à 110 et la valeur de b étant de 20 à 60 et a + b ≥ 80.

6. Composition de revêtement pelliculaire selon l'une des revendications précédentes, **caractérisée en ce qu'**elle contient du poloxamère 188, du poloxamère 237, du poloxamère 338 et/ou du poloxamère 407 ou un mélange de ceux-ci, le poloxamère 188 et/ou le poloxamère 237 étant particulièrement préférés.

7. Composition de revêtement pelliculaire selon l'une des revendications précédentes, **caractérisée en ce que** l'alcool polyvinylique (PVA) de formule générale II 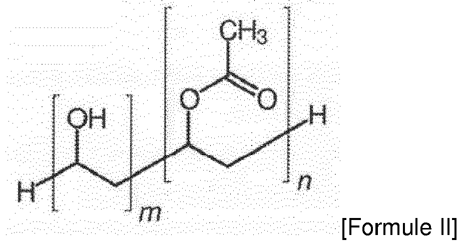 contient de l'acétate de polyvinyle partiellement hydrolysé, le rapport groupes acétyle (n)/groupes hydroxyle (m) étant n/m ≤ 0,30, de préférence situé dans la plage de 0,10 à 0,20, de manière particulièrement préférée dans la plage de 0,12 à 0,16.

8. Composition de revêtement pelliculaire selon l'une des revendications précédentes, **caractérisée en ce que** l'alcool polyvinylique (PVA) présente une viscosité de 3 à 10 mPa*s, de manière particulièrement préférée d'environ 4 à 6 mPa*s, en particulier d'environ 5 mPa*s, mesurée dans une solution à 4 %.

9. Composition de revêtement pelliculaire selon l'une des revendications précédentes, **caractérisée en ce que** l'alcool polyvinylique (PVA) est contenu selon une fraction pondérale de 15 % à 55 %, de préférence de 33 % à 47 %, rapportée au poids total de la composition de revêtement pelliculaire sèche.

10. Composition de revêtement pelliculaire selon l'une des revendications précédentes, **caractérisée en ce qu'**elle contient, en tant que charge, de la cellulose, du lactose, du carbonate de calcium, du kaolin et/ou du talc, selon une fraction pondérale de 10 % à 50 %, de préférence de 25 % à 40 %, rapportée au poids total de la composition de revêtement pelliculaire sèche.

11. Composition de revêtement pelliculaire selon l'une des revendications précédentes, **caractérisée en ce qu'**elle contient du poloxamère selon une fraction pondérale de 5 % à 35 %, de préférence de 8 % à 20 %, rapportée au poids total de la composition de revêtement pelliculaire sèche.

12. Composition de revêtement pelliculaire selon l'une des revendications précédentes, **caractérisée en ce qu'**elle contient un pigment ou un agent opacifiant, en particulier du dioxyde de titane ou de l'oxyde de fer, selon une fraction pondérale de 0 % à 40 %, de préférence de 5 % à 25 %, rapportée au poids total de la composition de revêtement pelliculaire sèche.

13. Composition de revêtement pelliculaire selon l'une des revendications précédentes, constituée de préférence
- d'alcool polyvinylique (PVA) selon une fraction pondérale de 30 % à 50 %,
- de talc selon une fraction pondérale de 20 % à 40 %,
- de poloxamère 188 ou de poloxamère 237 selon une fraction pondérale de 5 % à 20 %
- et, le cas échéant, de dioxyde de titane et/ou de pigments colorés selon une fraction pondérale de 5 % à 20 %,
les fractions pondérales étant rapportées au poids total de la composition de revêtement pelliculaire.

14. Substrat, en particulier médicament, complément alimentaire, denrée alimentaire, cosmétique, produit médical ou produit agricole, de préférence sous forme de comprimés, de pastilles, de granulés, de semences ou de cristaux, dont la surface extérieure est recouverte par la composition de revêtement pelliculaire selon l'une des revendications 1 à 13.

15. Dispersion de revêtement pelliculaire liquide destinée à être utilisée pour recouvrir des substrats, en particulier des médicaments, des compléments alimentaires, des denrées alimentaires, des cosmétiques, des dispositifs médicaux ou des produits agricoles, de préférence sous forme de comprimés, de pastilles, de granulés, de semences ou de cristaux, comprenant les composants de la composition de revêtement pelliculaire sèche selon l'une des revendications 1 à 13 dispersés et partiellement dissous dans un milieu liquide.

16. Dispersion de revêtement pelliculaire liquide selon la revendication 15, dispersée dans de l'eau, la fraction des composants non aqueux de la dispersion de revêtement pelliculaire étant comprise de préférence entre 10 et 30 % en poids, en particulier entre 15 % et 25 %, ou dispersée dans un mélange d'eau et d'alcool ayant une teneur en alcool de 1 à 50 % v/v, l'alcool étant de l'éthanol et/ou de l'isopropanol, et la fraction des composants non aqueux ou non alcooliques de la dispersion de revêtement pelliculaire étant comprise de préférence entre 10 et 30 % en poids, en particulier entre 15 % et 25 %.

17. Utilisation de polymères à blocs oxyde d'éthylène-oxyde de propylène ou poloxamères, présentant en particulier les caractéristiques selon l'une des revendications 1 à 6, en tant que plastifiant pour alcool polyvinylique (PVA), en particulier dans la fabrication d'un revêtement pelliculaire pour comprimés pharmaceutiques, à base d'alcool polyvinylique (PVA).

18. Procédé pour recouvrir des substrats, en particulier des médicaments, des compléments alimentaires, des denrées alimentaires, des cosmétiques, des dispositifs médicaux ou des produits agricoles, de préférence sous forme de comprimés, de pastilles, de granulés, de semences ou de cristaux, avec une pellicule, et qui comprend les étapes suivantes consistant à :
- mélanger les composants de la composition de revêtement pelliculaire sèche selon l'une des revendications 1 à 13 avec de l'eau et/ou de l'alcool pour former une dispersion ou une suspension de revêtement liquide,
- appliquer, de préférence pulvériser, une quantité efficace de cette dispersion sur les substrats pour former une pellicule sur la surface extérieure des substrats, et
- sécher la pellicule sur les substrats.
